# EUROPÄISCHE PATENTANMELDUNG

(11) **EP 2 078 947 A2**
(43) Veröffentlichungstag der Anmeldung: **15.07.2009**
(21) Anmeldenummer: 09150220.3
(22) Anmeldetag: 08.01.2009
(51) Int. Cl.: G01N 1/20

(54) **Messvorrichtung und Messverfahren zur automatisierten Messung der Eigenschaften des in einer Biogasanlage befindlichen Faulschlamms**

(30) Priorität: 14.01.2008 DE 102008004426
(71) Anmelder: ibk Bioanalytik GmbH, 86167 Augsburg (DE)
(72) Erfinder: Kottmair, Andreas, 86356, Neusäß (DE)
(74) Vertreter: Charrier, Rapp & Liebau

(57) **Zusammenfassung**

Die Erfindung betrifft eine Messvorrichtung (1) zur automatisierten Messung der Eigenschaften des in einer Prozessleitung (2) einer Biogasanlage befindlichen Faulschlamms (3), mit einem über eine Einlassöffnung (7) an die Prozessleitung (2) anschließbaren Messbehälter (5), einer Fördereinrichtung zum Befüllen und Entleeren des Messbehälters (5) mit einer Faulschlammprobe (11) aus dem in der Prozessleitung (2) befindlichen Faulschlamm (3), einem Prozessventil (14) zum Verschließen des Messbehälters (5), einer Einrichtung (16, 17, 18) zur Einleitung einer Reaktionsflüssigkeit (12) in den Messbehälter (5), einer Mischvorrichtung (22) zur Durchmischung der Faulschlammprobe (11) mit der eingeleiteten Reaktionsflüssigkeit (12), und einer Messeinheit (13) zur Bestimmung der Eigenschaften der Faulschlammprobe (11). Weiter betrifft die Erfindung ein entsprechendes Messverfahren mit einem an die Prozessleitung (2) über eine Einlassöffnung (7) anschließbaren Messbehälter (5), mit den folgenden Schritten: a) Befüllen des Messbehälters (5) mit einer Faulschlammprobe (11) aus dem in der Prozessleitung (2) befindlichen Faulschlamms (3), wobei zum Befüllen des Messbehälters (5) mit der Faulschlammprobe (11) in Schritt a) ein Unterdruck im Messbehälter (5) erzeugt wird, b) Trennung der Verbindung zwischen Messbehälter (5) und Prozessleitung (2), c) Einleiten einer Reaktionsflüssigkeit (12) in den verschlossenen Messbehälter (5), d) Durchmischen der Faulschlammprobe (11) mit der eingeleiteten Reaktionsflüssigkeit (12) durch Umwälzen eines im Messbehälter (5) befindlichen Gases, e) Messung der Eigenschaften der Faulschlammprobe (11) während oder nach Schritt d), f) Öffnen des Messbehälters (5), und g) Zurückfördern der Faulschlammprobe (11) in die Prozessleitung (2).

## Beschreibung

Die Erfindung betrifft eine Messvorrichtung und ein Messverfahren zur automatisierten Messung der Eigenschaften des in einer Biogasanlage befindlichen Faulschlamms.

Um bei Biogasanlagen die Prozessstabilität zu gewährleisten, müssen kontinuierlich die Eigenschaften des in der Biogasanlage und deren Prozessleitungen befindlichen Faulschlamms überwacht werden. Eine wichtige Eigenschaft ist die Konzentration des Hydrogencarbonats HCO₃⁻. Herkömmlich werden zur Messung der Hydrogencarbonat-Konzentration Faulschlammproben entnommen, welche dann im Labor überprüft werden. Anhand der Messergebnisse können dann die Betriebsparameter der Biogasanlage eingestellt werden. Das herkömmliche Verfahren ist umständlich und zeitaufwendig, da bei Abweichung der Hydrogencarbonat-Konzentration von den gewünschten Werten eine Nachstellung der Betriebsparameter erst mit großer zeitlicher Verzögerung erfolgen kann. Zudem erfordert die Probenentnahme den Einsatz von Personal, das hierdurch unnötigen Gefahren ausgesetzt wird.

Als Beispiel für eine bekannte Messvorrichtung offenbart die DE 198 52 397 A1 eine Vorrichtung zur Erfassung des anaeroben Abbaus in 0,5 bis 2 Liter-Reaktionsgefäßen als Kleinfaulturm in Form einer Laborapparatur. Diese Laborapparatur erlaubt es, in enger Anlehnung an die Verfahrensführung eines Faulturmes in biologischen Kläranlagen die sensiblen gekoppelten Abbaureaktionen durch spezielle Messsysteme wissenschaftlich vollständig zu erfassen. Bei dieser Vorrichtung kann zwar das Verhalten der in einem Faulturm enthaltenen Schlämme nachgebildet werden, es kann aber keine automatische Überwachung eines Faulturmes oder einer Biogasanlage durchgeführt werden.

Eine weitere bekannte Messvorrichtung geht aus der US 2007/0292308 A1 hervor. Diese offenbart einen Reaktionsbehälter, in den über eine Probenleitung und ein Mehrwegventil eine Flüssigkeitsprobe eingeleitet werden kann. Aus dem Reaktionsbehälter können dann Teile der Probenrückstände über eine Messeinrichtung und einen Überlaufbehälter wieder aus dem Reaktionsbehälter in die Probenleitung geleitet werden. Dieser Aufbau ist aufwändig und ermöglicht keine schnelle und einfache Messung einer Faulschlammprobe, da die Rückstände nicht ohne weiteres wieder aus dem Reaktionsbehälter entfernt werden können.

Eine weitere bekannte Messvorrichtung offenbart die DE 696 04 348 T2, bei der in einem Probeentnahmezylinder über ein Mengen-Probenentnahmekolbenpaar mit einem vorderen und hinteren Kolben eine Schlammprobe aus einer Schlammdurchführungspassage oder einem Schlammlagerungsbehälter entnommen werden kann, indem die Kolben gemeinsam Ausgefahren, dann wieder gemeinsam Eingefahren und anschließend der vordere Kolben gegen den feststehenden hinteren Kolben gezogen wird, um die Faulschlammprobe zu komprimieren und in einen Messbehälter zu fördern. Die dortige Anordnung der Mengen-Probenentnahmekolbenpaar im Probeentnahmezylinder ist kompliziert im Aufbau und das Einziehen und Abführen der Faulschlammprobe gestaltet sich zeitraubend und langwierig.

Deshalb besteht bei Biogasanlagen der Wunsch, die Eigenschaften des Faulschlamms vollautomatisch zu überwachen, was sich aber insbesondere aufgrund des hohen Trockensubstanzgehalts (> 5%) des Faulschlamms als schwierig erweist. Herkömmliche, im Labor verwendete Messvorrichtungen lassen sich aufgrund des hohen Trockensubstanzgehalts des Faulschlamms nicht ohne weiteres in Biogasanlagen einsetzen.

Der Erfindung liegt deshalb die Aufgabe zugrunde, die oben genannten Nachteile zu überwinden und eine Messvorrichtung und ein Messverfahren bereitzustellen, welche eine automatisierte, schnelle und einfache Messung der Eigenschaften des in einer Biogasanlage befindlichen Faulschlamms und eine schnelle und automatisierte Einwirkung auf die Prozessführung in der Biogasanlage ermöglichen.

Die Erfindung löst diese Aufgabe durch eine Messvorrichtung mit den Merkmalen des Anspruchs 1 und ein Messverfahren mit den Merkmalen des Anspruchs 11. Vorteilhafte Weiterbildungen und Ausgestaltungen der Erfindung sind in den Unteransprüchen angegeben.

Die erfindungsgemäße Messvorrichtung und das erfindungsgemäße Messverfahren ermöglichen es, in einem automatisierten Arbeitsgang sowohl eine Entnahme einer Faulschlammprobe aus der Biogasanlage als auch die Bestimmung der Eigenschaften der Faulschlammprobe durchzuführen, wobei der Faulschlamm bzw. die Faulschlammprobe hierbei auch einen vergleichsweise hohen Trockensubstanzgehalt > 5% aufweisen kann.

In einer zweckmäßigen Ausführung sieht die Erfindung vor, dass die Mischvorrichtung eine Mischpumpe umfasst, die saugseitig an einen mit Gas gefüllte ersten Bereich und förderseitig an einen mit der Faulschlammprobe gefüllten zweiten Bereich des Messbehälters angeschlossen ist, um zur Durchmischung der im Messbehälter befindlichen Faulschlammprobe mit der eingeleiteten Reaktionsflüssigkeit das Gas einfach und schnell umwälzen zu können. Um die Faulschlammprobe vor dem Einleiten der Reaktionsflüssigkeit mit Gas, insbesondere gasförmigem Kohlendioxid sättigen zu können, sieht eine vorteilhafte Ausführung der Erfindung vor, dass die Mischpumpe saugseitig über ein Sättigungsventil mit einem mit Gas gefüllten dritten Bereich des Messbehälters verbunden sein kann.

Für den automatisierten Betrieb kann die Fördereinrichtung vorteilhaft einen innerhalb des Messbehälters verschiebbaren und abdichtenden Kolben aufweisen. Um Reste einer bereits verwendeten Faulschlammprobe aus dem Messbehälter besser entfernen zu können und somit die Ergebnisse nachfolgender Messungen zu verbessern, enthält der Kolben eine abdichtend an einer Innenwand des Messbehälters anliegende Dichtung, z.B. eine Dichtlippe, die in einer vorteilhaften Ausführung vom Kolben zur Messöffnung hin schräg nach außen zur Innenwand hin verläuft. Die Dichtung verringert zudem die Gefahr, dass ein während der Durchmischung entstehender Überdruck aus dem Messzylinder am Kolben vorbei entweichen kann, was ebenfalls nachteilig für das Messergebnis wäre. Vielmehr stellt die spezielle Ausgestaltung der Dichtung sicher, dass mit ansteigendem Druck eine bessere Abdichtung erreicht wird. Hierdurch können möglichst gute Messergebnisse für wiederholte automatische Messvorgänge sichergestellt werden.

Die Erfindung wird nun anhand von Ausführungsbeispielen mit Bezug auf die beiliegende Zeichnungen beschrieben. Diese zeigen:
- **Fig. 1**: ein Blockschaltbild einer an eine Prozessleitung einer Biogasanlage angeschlossenen erfindungsgemäßen Messvorrichtung;
- **Fig. 2**: ein Blockschaltbild einer alternative Ausführungsform einer erfindungsgemäßen Messvorrichtung;
- **Fig. 3 - 6**: eine Ablaufbeschreibung des erfindungsgemäßen Messverfahrens anhand schematischer Darstellungen der Messvorrichtung aus Fig. 1;
- Fig. 7: eine Zylinder-Kolben-Einheit der erfindungsgemäßen Vorrichtung im Detail als Schnittdarstellung;
- **Fig. 8**: ein Blockschaltbild einer alternative Ausführungsform einer erfindungsgemäßen Messvorrichtung.

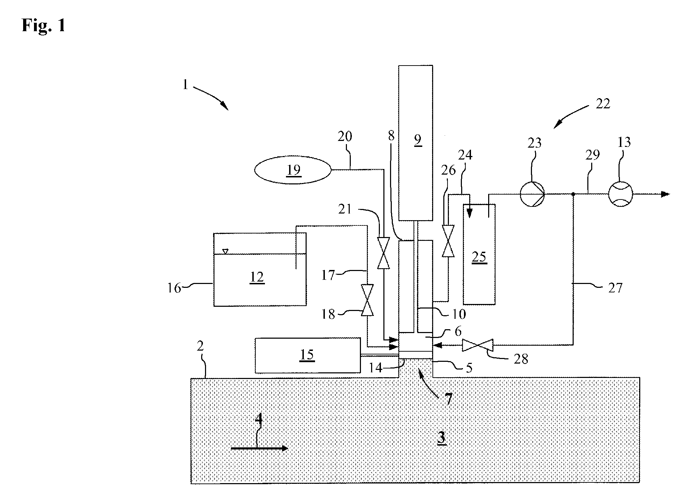

Eine in Fig. 1 dargestellte erfindungsgemäße Messvorrichtung 1 ist an eine Prozessleitung 2 angeschlossen, welche zwei zeichnerisch nicht dargestellte Faultürme einer Biogasanlage zum Austausch von Faulschlamm 3 in eine Flussrichtung 4 verbindet. Der Faulschlamm 3 liefert beständig geeignetes Probenmaterial aus dem Inneren der Faultürme, solange der darin befindliche Faulschlamm durchmischt wird. Gegebenenfalls kann die Messvorrichtung 1 auch direkt an einem Faulturm angeordnet werden.

Kernstück der Messvorrichtung 1 ist eine als an sich bekannte Zylinder-Kolbenpumpe ausgebildete Fördereinrichtung zum Befüllen und Entleeren eines Messbehälters, die einen als Messzylinder 5 ausgebildeten Messbehälter und einen darin verschiebbar und abdichtend an einer Innenwand des Messzylinders 5 anliegenden Kolben 6 aufweist.

Der Messzylinder 5 ist im wesentlichen lotrecht stehend mit einer nach unten weisenden Einlassöffnung 7 an die Prozessleitung 2 angeschlossen. An dem in den Zeichnungen oberen Ende 8 des Messzylinders 5 ist ein Kolbenantrieb 9 angeordnet, welcher den Kolben 6 über eine Antriebsstange 10 zwischen einer Anfangsstellung in der Nähe der Einlassöffnung 7 und einer oberen Endstellung in der Nähe des oberen Endes 8 des Messzylinders 5 hin- und her bewegen kann. Der Kolbenantrieb 9 wird vorliegend hydraulisch betätigt, kann aber ebenso pneumatisch oder elektromotorisch betrieben werden.

Im vorliegenden Ausführungsbeispiel wird mit Hilfe Messvorrichtung 1 die Konzentration der in einer aus dem Faulschlamm 3 der Prozessleitung 2 gezogenen Faulschlammprobe 11 enthaltenen Hydrogencarbonats gemäß der folgenden chemischen Reaktion bestimmt:

Hydrogencarbonat + Säure → Kohlendioxid + Wasser + Säureanion

Wird als Säure wasserfreie Salzsäure (HCl) verwendet, ergibt sich die folgende Gleichung:

1 HCO₃⁻ + 1 HCl → 1 CO₂ + 1 H₂O + 1 Cl⁻

Durch das Zusetzen der Salzsäure wird das in der Faulschlammprobe 11 gelöste Hydrogencarbonat als gasförmiges Kohlendioxid im Messzylinder 5 freigesetzt und anschließend mittels einer als Gasmengenerfassungsvorrichtung ausgebildeten Messeinheit 13 zur Bestimmung der Eigenschaften der im Messzylinder 5 befindlichen Faulschlammprobe 11 gemessen. Anhand der im Messzylinder 5 freigesetzten und in die Messeinheit 13 geleiteten Kohlendioxidmenge kann dann mit obiger Gleichung die Menge des in der Faulschlammprobe 11 enthaltenen Hydrogencarbonats bestimmt werden. Grundsätzlich müssen noch die aufgrund der durch die Löslichkeit von Kohlendioxid in der Faulschlammprobe 11 auftretenden Verluste berücksichtigt werden, wobei die physikalisch gelöste Menge an Kohlendioxid in der Faulschlammprobe 11 proportional zum Kohlendioxid-Partialdruck in der umgebenden Atmosphäre ist (Henry-Gesetz).

Um für die Messung an der Faulschlamprobe 11 im Messzylinder 5 ein definiertes Volumen einstellen zu können, in dem die oben genannte Reaktion unter definierten Bedingungen ablaufen kann, sieht die Messvorrichtung 1 ein Prozessventil 14 vor, welches die Einlassöffnung 7 des Messzylinders 5 verschließen bzw. öffnen kann. Das Prozessventil 14 ist vorliegend ein Schieberventil, welches über einen Stellantrieb 15 automatisch geöffnet und geschlossen werden kann. Alternativ kann auch vorteilhaft ein Kugelhahnventil oder ein anderes geeignetes Ventil eingesetzt werden.

Zum Einleiten der für obige Reaktion benötigten Reaktionsflüssigkeit 12, hier Salzsäure, in einen Bereich des Messzylinders 5 zwischen Kolben 6 und geschlossenem Prozessventil 14 ist eine Einrichtung zur Einleitung der Reaktionsflüssigkeit vorgesehen. Diese enthält einen Reaktionsflüssigkeitsvorrat 16, aus dem über eine Reaktionsflüssigkeitsleitung 17 und ein automatisch betreibbares Reaktionsflüssigkeitsventil 18 die Reaktionsflüssigkeit 12 in den geschlossenen Messzylinder 5 eingeleitet werden kann.

Weiter weist die Messvorrichtung 1 einen Spülgasvorrat 19 auf, aus welchem über eine Spülgaszuleitung 20 und ein Spülgasventil 21 ein Spülgas in den Messzylinder 5 geleitet werden kann. Das Spülgas ist im vorliegenden Ausführungsbeispiel Kohlendioxid, es können aber ebenso Stickoxid oder andere geeignete Gase verwendet werden.

Um die im Messzylinder 5 enthaltene Faulschlammprobe 11 mit der eingeleiteten Salzsäure vermischen zu können, sieht die Messvorrichtung 1 eine Mischvorrichtung 22 vor. Die Mischvorrichtung 22 umfasst eine Mischpumpe 23 für Gas, welche saugseitig über eine Entnahmeleitung 24 an einen in Fig. 1 oberen Bereich des Messzylinders 5 angeschlossen ist. In der Entnahmeleitung 24 ist vor dem Eingang der Mischpumpe 23 ein Entschäumer 25 sowie ein automatisch betreibbares Entnahmeventil 26 angeordnet, mit dem die Entnahmeleitung 24 abgesperrt werden kann. Der Entschäumer 25 dient zum Schutz der Mischpumpe 23. Förderseitig ist die Mischpumpe 23 über eine Mischleitung 27 an einen in Fig. 1 unteren Bereich des Messzylinders 5 angeschlossen, welche über ein automatisch betreibbares Mischventil 28 gesperrt werden kann.

Zum Messen der gemäß obiger Gleichung im Messzylinder 5 freigesetzten Kohlendioxidmenge ist die Messeinheit 13 in einer in Strömungsrichtung nach der Mischpumpe 23 abzweigenden Messleitung 29 angeordnet.

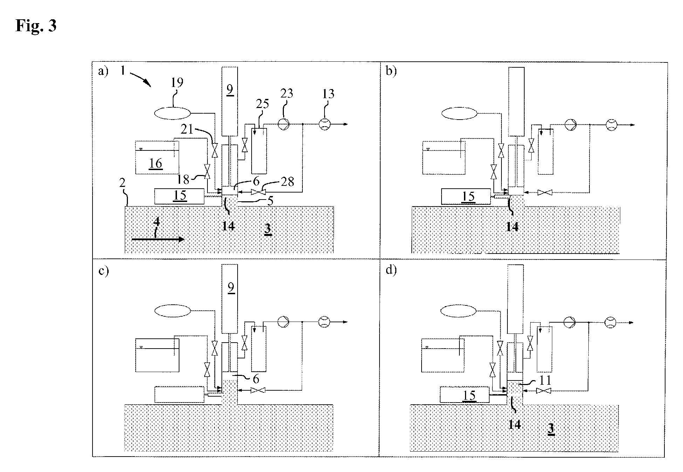

Die Funktionsweise der Messvorrichtung 1 wird nun anhand der Fig. 3 bis 6 ausführlich erläutert. Aus Gründen der besseren Übersichtlichkeit werden in den Fig. 3 bis 6 lediglich Bezugszeichen für die im jeweiligen Verfahrensschritt maßgeblichen Bestandteile der Messvorrichtung 1 verwendet, die übrigen Bezugszeichen lassen sich ohne weiteres aus Fig. 1 entnehmen.

In Fig. 3 a) ist der Ausgangszustand der Messvorrichtung 1 vor einer Messung gezeigt. Das Prozessventil 14 ist geschlossen und der Kolben 6 befindet sich in seiner in Fig. 1 untersten Stellung. Das Reaktionsflüssigkeitsventil 18, das Spülgasventil 21, das Entnahmeventil 26 und das Mischventil 28 sind geschlossen, die Mischpumpe 23 außer Betrieb. Gemäß Fig. 3 b) wird in einem ersten Schritt das Prozessventil 14 durch den Stellantrieb 15 geöffnet, so dass der Messzylinder 5 mit Faulschlamm 3 befüllt werden kann. Anschließend wird gemäß Fig. 3 c) der Kolben 6 durch den Kolbenantrieb 9 um eine vorgegebene Länge nach oben bewegt, um im Messzylinder 5 einen Unterdruck zu erzeugen und eine definierte Menge an Faulschlamm 3, also die Faulschlammprobe 11, in den Messzylinder 5 zu saugen. Danach wird gemäß Fig. 3 d) das Prozessventil 14 wieder geschlossen.

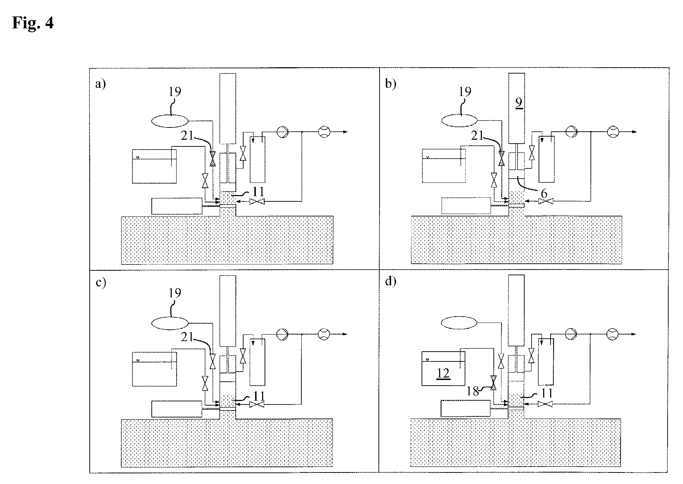

In einem nächsten Schritt wird gemäß Fig. 4 a) das Spülgasventil 21 geöffnet und gemäß Fig. 4 b) der Kolben 6 um eine vorgegebene Strecke nach oben gefahren, um mittels Unterdruck eine vorgegebene Menge Spülgas aus dem Spülgasvorrat 19 in den Messzylinder 5 zu saugen. Nachfolgend wird gemäß Fig. 4 c) das Spülgasventil 21 wieder geschlossen. Das Spülgas im Messzylinder 5 dient dazu, über der Faulschlammprobe 11 einen gasgefüllten Bereich zu schaffen, dessen Funktion später erläutert wird.

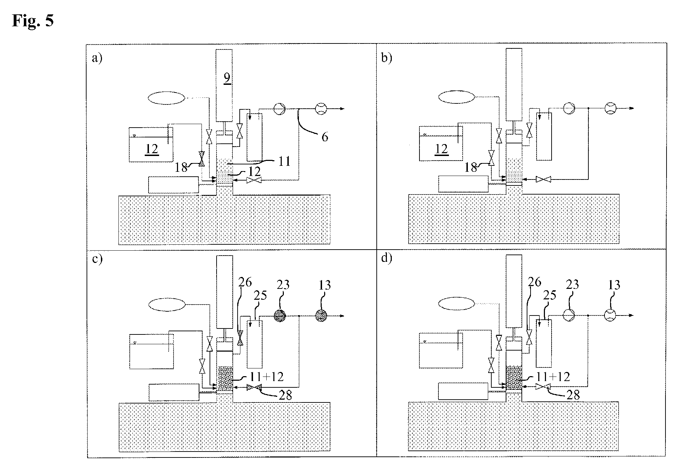

Als nächster Schritt wird gemäß Fig. 4 d) das Reaktionsflüssigkeitsventil 18 geöffnet. Durch ein weiteres Verfahren des Kolbens 6 von der in Fig. 4 d) gezeigten Stellung um eine vorgegeben Strecke in die in Fig. 5 a) gezeigte Stellung wird wiederum ein Unterdruck im Messzylinder 5 erzeugt, durch den eine vorgegebene Menge an Salzsäure aus dem Reaktionsflüssigkeitsvorrat 16 in den Messzylinder 5 gesaugt wird. Anschließend wird gemäß Fig. 5 b) das Reaktionsflüssigkeitsventil 18 wieder geschlossen, während der Kolben 6 in seiner oberen Endstellung verbleibt.

Um die oben beschriebene Reaktion in Gang zu setzen, muss die Faulschlammprobe 11 mit der Salzsäure vermischt werden, wozu die Mischvorrichtung in Betrieb genommen wird. Hierzu werden gemäß Fig. 5 c) das Entnahmeventil 26 und das Mischventil 28 geöffnet und die Mischpumpe 23 in Betrieb genommen, so dass Spülgas aus dem oberen Bereich des Messzylinders 5 in den unteren, die Faulschlammprobe 11 und die Salzsäure enthaltenden Bereich des Messzylinders 5 umgewälzt wird. Das gemäß obiger Reaktion entstehende Kohlendioxid erzeugt einen Überdruck im Messzylinder 5 und der Mischvorrichtung 22 und gelangt zur Messeinheit 13. Die Durchmischung wird fortgesetzt, bis die Reaktion abgeschlossen und sämtliches Hydrogencarbonat in der Faulschlammprobe 11 umgesetzt ist. Dann wird kein Kohlendioxid und folglich kein Überdruck mehr erzeugt, so dass aus der in der Messeinheit 13 gemessenen Kohlendioxidmenge die Hydrogencarbonat-Konzentration bestimmt werden kann. Durch Überwachung des Drucks kann das Abfallen des Überdrucks gemessen werden, worauf dann gemäß Fig. 5 d) die Mischpumpe 23 abgeschaltet und das Entnahmeventil 26 und das Mischventil 28 geschlossen werden. Alternativ kann dies auch aufgrund fehlender Messwertänderungen in der Messeinheit 13 oder nach einer vorgegebenen Zeit erfolgen.

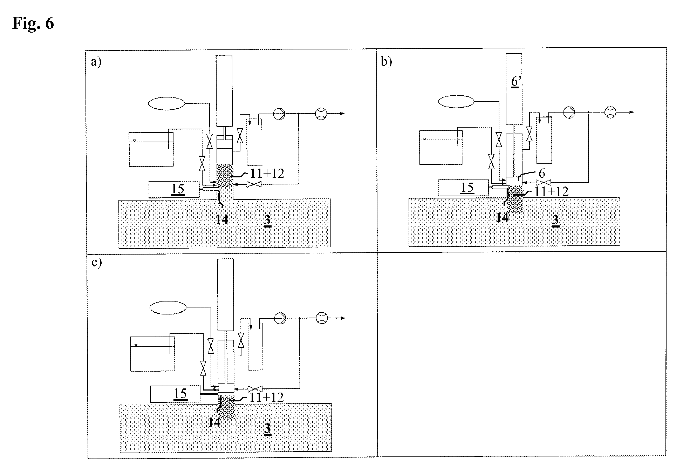

Um den Messzylinder 5 für die nächste Messung vorzubereiten, wird anschließend gemäß Fig. 6 a) das Prozessventil 14 wieder geöffnet und der Kolben 6 von seiner oberen Endstellung in Fig. 6 a) in seine untere Endstellung in Fig. 6 b) bewegt. Die Faulschlammprobe 11 wird hierdurch wieder in die Prozessleitung 2 zurückgefördert. Da bei der Reaktion der Faulschlammprobe 11 mit der Salzsäure keine gefährlichen Produkte entstehen, kann der Analysenrückstand der Faulschlammprobe 11, also angesäuerter Faulschlamm, ohne weiteres wieder in den Faulschlamm 3 entleert werden, was einen zusätzlichen Aufwand für die Entsorgung des Analysenrückstands unnötig macht. Anschließend wird gemäß Fig. 6 c) das Prozessventil 14 wieder geschlossen, so dass wieder der in Fig. 3 a) dargestellte Ausgangszustand der Messvorrichtung 1 erreicht ist und eine neue Messung durchgeführt werden kann.

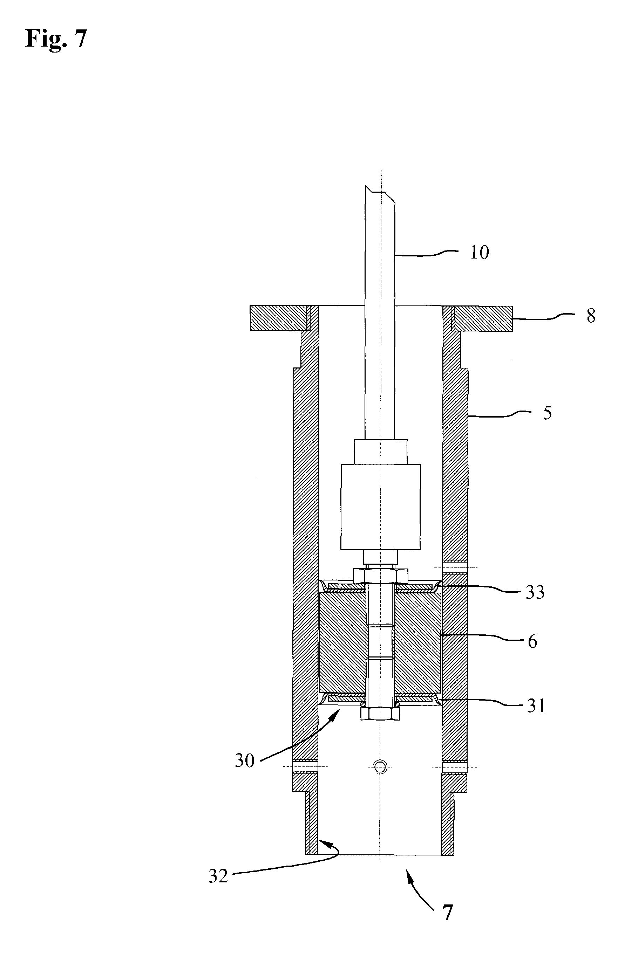

Der Kolben 6 weist gemäß Fig. 7 an seiner der Einlassöffnung 7 zugewandten Vorderseite 30 eine umlaufende Dichtung 31 auf, welche abdichtend an einer Innenwand 32 des Messbehälters 5 anliegt und vom Kolben 6 zur Einlassöffnung 7 hin schräg nach außen zur Innenwand 32 hin verläuft. Hierdurch wird sichergestellt, dass während der laufenden Reaktion gemäß Fig. 5 c) im Messzylinder 5 kein Kohlendioxid am Kolben 6 vorbei entweicht, sondern das gesamte erzeugte Kohlendioxid über die Entnahmeleitung 24, den Entschäumer 25, die Mischpumpe 23 und die Messleitung 29 zur Messeinheit 13 gelangt. Besonders bei zunehmendem Überdruck wird die speziell ausgebildete Dichtlippe 31 immer stärker an die Innenwand 32 des Messzylinders 5 gedrückt, so dass die Abdichtung mit höherem Druck noch verstärkt wird. Gleichzeitig dient die Dichtung 31 dazu, gemäß Fig. 6 a bis c) die Reaktionsreste der Faulschlammprobe 11 vollständig aus dem Messzylinder 5 zu entfernen. Eine weitere, in Aufbau und Funktion der Dichtung 31 entsprechende Dichtung 33 an der Kolbenrückseite verhindert zusätzliche Undichtigkeiten und das Anhaften von Faulschlammresten beim Bewegen des Kolbens 6 nach oben.

Bei der oben beschriebenen Ausführung ist es vorteilhaft, wenn der Messzylinder 5 im wesentlichen lotrecht über der Prozessleitung 2 angebracht ist. In einer alternativen Ausführung kann durch geringfügige Anpassungen die Messeinrichtung 1 aber auch in einer anderen räumlichen Lage verwendet werden. Beispielsweise kann die Messeinrichtung 1 auch nach unten hängend an der Prozessleitung 2 angeordnet werden. Um dann den Messzylinder 5 mit Faulschlamm 3 zu befüllen, wird der Kolben 6 soweit zur Einlassöffnung 7 hin nach oben gefahren, dass durch die Schwerkraft bedingt nur die vorgegebene Menge an Faulschlamm 3, also die Faulschlammprobe 11, in den Messzylinder 1 fällt bzw. fließt. Hierzu müssen lediglich die Anschlüsse der Entnahmeleitung 24 und der Mischleitung 27 vertauscht werden, damit das sich dann im oberen Bereich des Messzylinders 5 sammelnde Gas in den unteren, die Faulschlammprobe 11 und die Salzsäure enthaltenden Bereich des Messzylinders 5 umgewälzt werden kann.

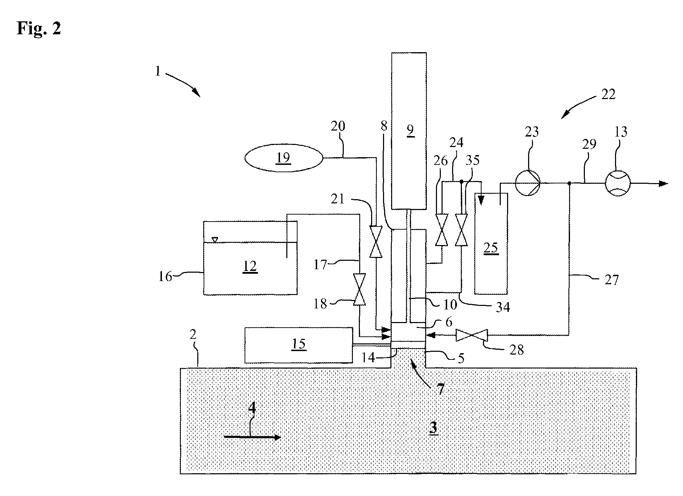

Wie oben dargelegt, ist es für die Bestimmung der Hydrogencarbonat-Konzentration vorteilhaft, wenn die Faulschlammprobe 11 vor Beginn der Reaktion mit Kohlendioxid gesättigt ist. Denn dann entspricht das gesamte in der Messeinheit 13 gemessene Kohlendioxid dem in der Faulschlammprobe 11 enthaltenen und während der Reaktion umgesetzten Hydrogencarbonat. Dies kann durch die in Fig. 2 dargestellte alternative Ausführung der erfindungsgemäßen Messvorrichtung sichergestellt werden. Diese unterscheidet sich von der in Fig. 1 dargestellten Ausführung lediglich durch eine zusätzliche Sättigungsleitung 34, welche von einem mittleren Bereich des Messzylinders 5 zum Entschäumer 25 führt und mit einem automatisch betriebenen Sättigungsventil 35 abgesperrt werden kann. Hierdurch kann die Faulschlammprobe 11 bereits während der gemäß Fig. 4 a) und b) dargestellten Einleitung des Sauggases in den Messzylinder 5, also vor dem Einleiten der Salzsäure, über die Sättigungsleitung 34, den Entschäumer 25, die Mischpumpe 23 und die Mischleitung 27 mit Sauggas, hier Kohlendioxid, gesättigt werden.

Durch die automatische Ansteuerung des Kolbens 6, der Mischpumpe 23 und sämtlicher Ventile 14, 18, 21, 26, 28 und 35 der Messvorrichtung 1 sowie gegebenenfalls der Überwachung des Drucks in der Mischvorrichtung 22 und im Messzylinder 5 kann die Messeinrichtung erfindungsgemäß vollautomatisch betrieben werden, ohne dass manuelle Entnahmen oder Eingriffe notwendig sind. Hierdurch wird die Überwachung und Steuerung der Betriebsprozesse der Biogasanlage deutlich vereinfacht. Zudem ermöglicht die automatische Messung eine relativ hohe Messfrequenz, beispielsweise zwei Messungen pro Tag, die unter genau vorgegebenen Bedingungen durchgeführt werden können. Eine zeitraubende manuelle Probenentnahme mit nachfolgender Untersuchung im Labor ist nicht mehr notwendig.

Zusätzlich kann vorteilhaft ein in den Ausführungsbeispielen nicht zeichnerisch dargestellter Spülwasserzugang am Messzylinder 5 angeordnet sein, über den manuell oder automatisch unter hohem Druck Spülwasser in den Messzylinder 5 geleitet werden kann, um diesen zu Reinigen oder eine Blockierung des Prozessventils 14 aufzuheben. Weiter kann vorteilhaft ein nicht zeichnerisch dargestellter Zugang im Messzylinder 5 vorgesehen sein, über den eine Standardlösung manuell oder automatisch zugegeben und dann anstelle der Faulschlammprobe 11 entsprechend der in den Fig. 4 und 5 gezeigten Schritte analysiert werden kann. Hierdurch kann die Messeinheit justiert bzw. Fehlmessungen erkannt werden.

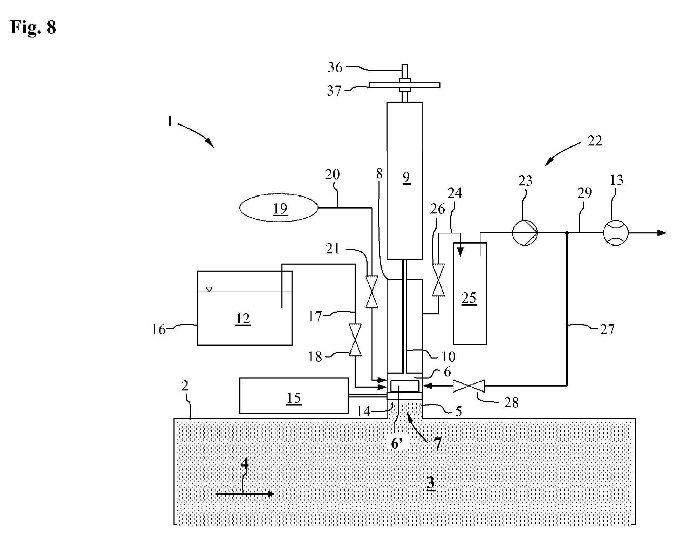

Die in Fig. 8 dargestellte weitere alternative Ausführung der erfindungsgemäßen Messvorrichtung unterscheidet sich von der in Fig. 1 dargestellten im wesentlichen durch einen im Kolben 6 angeordneten Ultraschallerzeuger 6' sowie eine detailliert dargestellte Vorschubeinstelleinrichtung für den Kolben samt Antrieb.

Der Ultraschallerzeuger 6' ist in dem Bereich des Kolbens 6 angeordnet, der der Faulschlammprobe 11 zugewandt ist. Durch den Ultraschallerzeuger 6' wird die eingesogene Faulschlammprobe 11 mit Ultraschall beschallt und hierdurch mit der in den verschlossenen Messbehälter 5 eingeleiteten Reaktionsflüssigkeit 12 vermischt. Der Einsatz des Ultraschallerzeugers 6' als Mischvorrichtung verringert die zur Durchführung des erfindungsgemäßen Messverfahrens notwendige Zeit deutlich, so dass eine schnellere Messung möglich ist. Hierdurch kann zudem vorteilhaft die Anzahl von Messungen je Zeiteinheit erhöht werden. Der Messzylinder 5 ist bei dieser Ausführung vorteilhaft nach unten hängend mit nach oben weisender Einlassöffnung 7 an der Prozessleitung 2 angeordnet, um einen für die Ultraschallübertragung möglichst guten Kontakt mit der Faulschlammprobe 11 zu gewährleisten. Auch bei dieser hängenden Anordnung wird die Faulschlammprobe 11 mit Unterdruck aus der Prozessleitung 2 in den Messzylinder 5 gesaugt.

Um das Volumen des Messraums innerhalb des Messzylinders 5 möglichst einfach und dennoch genau einstellen zu können, ist die aus Kolben 6, Antriebsstange 10 und Kolbenantrieb 9 gebildete Kolben-Antriebs-Einheit über eine als Spindeltrieb 36, 37 ausgebildete Vorschubeinstelleinrichtung vorteilhaft an einem nicht gezeigten ortsfesten Träger angeordnet. Durch den Spindeltrieb 36, 37 kann der Kolbenantrieb 9 in Vorschubrichtung des Kolbens 6 zwischen Träger und Messzylinder 5 bewegt werden, so dass der Abstand zwischen geschlossenem Prozessventil 14 und dem Kolben 6 in der oberen Endstellung und somit das Volumen des Messraums im Messzylinder 5 sehr fein justiert werden können. Somit können auch bei fest eingestelltem Vorschub bzw. Anfangs- und Endstellung des Kolbens 6 unterschiedlich große Volumina des Messraums im geschlossenen Messzylinder 5 eingestellt werden, ohne den Vorschub des Kolbens 6 ändern zu müssen. Anstelle des Spindeltriebs 36, 37 können auch andere Vorschubmittel verwendet werden.

## Patentansprüche

1. Messvorrichtung (1) zur automatisierten Messung der Eigenschaften des in einer Prozessleitung (2) einer Biogasanlage befindlichen Faulschlamms (3), mit
- einem über eine Einlassöffnung (7) an die Prozessleitung (2) anschließbaren Messbehälter (5),
- einer Fördereinrichtung zum Befüllen und Entleeren des Messbehälters (5) mit einer Faulschlammprobe (11) aus dem in der Prozessleitung (2) befindlichen Faulschlamm (3),
- einem Prozessventil (14) zum Verschließen des Messbehälters (5),
- einer Einrichtung (16, 17, 18) zur Einleitung einer Reaktionsflüssigkeit (12) in den Messbehälter (5),
- einer Mischvorrichtung (6', 22) zur Durchmischung der Faulschlammprobe (11) mit der eingeleiteten Reaktionsflüssigkeit (12), und
- einer Messeinheit (13) zur Bestimmung der Eigenschaften der Faulschlammprobe (11).

2. Messvorrichtung (1) nach Anspruch 1, **dadurch gekennzeichnet, dass** dem Messbehälter (5) eine Spülgaszuführung (19, 20 21) zum Einleiten von Spülgas zugeordnet ist.

3. Messvorrichtung (1) nach Anspruch 1 oder 2, **dadurch gekennzeichnet, dass** die Mischvorrichtung (22) eine Mischpumpe (23) umfasst, die saugseitig an einen mit Gas gefüllten ersten Bereich und förderseitig an einen mit der Faulschlammprobe (11) gefüllten zweiten Bereich des Messbehälters (5) angeschlossen ist.

4. Messvorrichtung (1) nach Anspruch 3, **dadurch gekennzeichnet, dass** die Mischpumpe (23) saugseitig über ein Entnahmeventil (26) mit dem ersten Bereich des Messbehälters (5) und/oder förderseitig über ein Mischventil (28) mit dem zweiten Bereich des Messbehälters (5) verbunden ist und/oder saugseitig über ein Sättigungsventil (35) mit einem mit Gas gefüllten dritten Bereich des Messbehälters (5) verbunden ist.

5. Messvorrichtung (1) nach einem der voranstehenden Ansprüche, **dadurch gekennzeichnet, dass** das Prozessventil (14) ein mittels eines Stellantriebes betätigbares Schieberventil oder Kugelhahnventil ist.

6. Messvorrichtung (1) nach einem der voranstehenden Ansprüche, **dadurch gekennzeichnet, dass** der Messbehälter (5) im wesentlichen lotrecht mit nach unten weisender Einlassöffnung (7) an der Prozessleitung (2) angeordnet ist.

7. Messvorrichtung (1) nach einem der Ansprüche 1 bis 5, **dadurch gekennzeichnet, dass** der Messbehälter (5) nach unten hängend mit nach oben weisender Einlassöffnung (7) an der Prozessleitung (2) angeordnet ist.

8. Messvorrichtung (1) nach einem der voranstehenden Ansprüche, **dadurch gekennzeichnet, dass** die Fördereinrichtung einen innerhalb des Messbehälters (5) verschiebbaren Kolben (6) aufweist.

9. Messvorrichtung (1) nach Anspruch 8, **dadurch gekennzeichnet, dass** im Kolben (6) ein Ultraschallerzeuger (6') vorgesehen ist.

10. Messvorrichtung (1) nach einem der Ansprüche 8 oder 9, **dadurch gekennzeichnet, dass** die Fördereinrichtung den Kolben (6) und einen diesem zugeordneten Kolbenantrieb (9) umfasst, wobei der Kolbenantrieb (9) über eine Vorschubeinstelleinrichtung (36, 37) in Kolbenvorschubrichtung zwischen einem ortsfesten Träger und dem Messbehälter (5) bewegbar ist.

11. Messverfahren zur automatisierten Messung der Eigenschaften des in einer Prozessleitung (2) einer Biogasanlage befindlichen Faulschlamms (3), mit einem an die Prozessleitung (2) über eine Einlassöffnung (7) anschließbaren Messbehälter (5), mit den folgenden Schritten:
a) Befüllen des Messbehälters (5) mit einer Faulschlammprobe (11) aus dem in der Prozessleitung (2) befindlichen Faulschlamms (3), wobei zum Befüllen des Messbehälters (5) mit der Faulschlammprobe (11) in Schritt a) ein Unterdruck im Messbehälter (5) erzeugt wird,
b) Trennung der Verbindung zwischen Messbehälter (5) und Prozessleitung (2),
c) Einleiten einer Reaktionsflüssigkeit (12) in den verschlossenen Messbehälter (5),
d) Durchmischen der Faulschlammprobe (11) mit der eingeleiteten Reaktionsflüssigkeit (12) durch Umwälzen eines im Messbehälter (5) befindlichen Gases,
e) Messung der Eigenschaften der Faulschlammprobe (11) während oder nach Schritt d),
f) Öffnen des Messbehälters (5), und
g) Zurückfördern der Faulschlammprobe (11) in die Prozessleitung (2).

12. Messverfahren nach Anspruch 11, **dadurch gekennzeichnet, dass** zwischen Schritt b) und c) ein Spülgas, insbesondere Kohlendioxid oder Stickoxid, in den Messbehälter (5) eingeleitet wird und/oder dass in Schritt d) Gas aus einem mit Gas gefüllten ersten Bereich des Messbehälters (5) abgeleitet und in einen mit der Faulschlammprobe (11), Reaktionsflüssigkeit (12) und/oder Faulschlammproben-Reaktionsflüssigkeits-Gemisch gefüllten zweiten Bereich des Messbehälters (5) zur Durchmischung der Faulschlammprobe (11) mit der Reaktionsflüssigkeit (12) eingeleitet wird.

13. Messverfahren nach einem der Ansprüche 11 bis 12, **dadurch gekennzeichnet, dass** zum Einleiten der Reaktionsflüssigkeit (12) aus einem Reaktionsflüssigkeitsvorrat (16) in Schritt c) und/oder zum Einleiten des Spülgases aus einem Spülgasvorrat (19) ein Unterdruck im Messbehälter (5) erzeugt wird.

14. Messverfahren nach einem der Ansprüche 11 bis 13, **dadurch gekennzeichnet, dass** der Unterdruck im Messbehälter (5) durch abschnittsweises Verschieben eines im Messbehälter (5) zwischen einer einer Einlassöffnung (7) des Messbehälters (5) näheren Anfangsstellung und einer Endstellung verschiebbaren Kolbens (6) erzeugt wird.

15. Messverfahren nach einem der Ansprüche 11 bis 14, **dadurch gekennzeichnet, dass** die Faulschlammprobe (11) in Schritt d) mit Ultraschall beschallt wird.
